# EUROPÄISCHE PATENTSCHRIFT

(11) **EP 1 032 109 B1**
(45) Veröffentlichungstag und Bekanntmachung des Hinweises auf die Patenterteilung: **15.09.2004**
(21) Anmeldenummer: 99122695.2
(22) Anmeldetag: 15.11.1999
(51) Int. Cl.: H02J 7/02, A61N 1/378, H04R 25/00

(54) **Vorrichtung und Verfahren zum Unterstützen der Positionierung eines externen Sendeteils mit Bezug auf ein implantierbares Empfangsteil eines Ladesystems eines implantierbaren medizinischen Gerätes**
Method and device to assist the positioning of an external transmitting part relating to an implantable receiving part of an implantable medical devices's charging system
Procédé et dispositif d'aide au positionnement d'une élément émetteur externe,par rapport à un élément récepteur implantable d'une système de charge d'appareils médicaux implantables

(30) Priorität: 26.02.1999 DE 19908438
(43) Veröffentlichungstag der Anmeldung: 30.08.2000
(73) Patentinhaber: Cochlear Limited, Lane Cove, NSW 2066 (AU)
(72) Erfinder: Hein, Walter, 82110 Germering (DE); Mayer, Reinhard, 81667 München (DE)
(74) Vertreter: Schwan, Gerhard, Dipl.-Ing.

(56) Entgegenhaltungen:
- US-A- 5 279 292
- US-A- 5 690 693
- US-A- 5 814 095
- P:E:K: DONALDSON: "Power For Neurological Prostheses: A Simple Inductive R.F. Link With Improved Performance" BIOMEDICAL ENGINEERING, Bd. 9, Nr. 3, 3. Juli 1987 (1987-07-03), Seiten 194-197, XP009007923

## Beschreibung

Die vorliegende Erfindung betrifft eine Vorrichtung gemäß dem Oberbegriff von Anspruch 1. Die Erfindung befaßt sich ferner mit einem Verfahren zum Unterstützen der Positionierung eines externen Sendeteils mit Bezug auf ein implantierbares Empfangsteil eines Ladesystems zum Aufladen einer wiederaufladbaren Energiequelle eines implantierbaren medizinischen Gerätes, insbesondere einer implantierbaren Hörhilfe, wobei der Sende- und der Empfangsteil jeweils einen Resonanzkreis mit einer Sendespule beziehungsweise einer Empfangsspule aufweisen und wobei diese Spulen durch entsprechendes manuelles Positionieren des Sendeteils für eine transkutane Energieübertragung induktiv miteinander gekoppelt werden.

Eine Vorrichtung und ein Verfahren dieser Art sind aus US-PS 5 279 292 bekannt. Dabei ist einer Ladeelektronik des Empfangsteils eine Telemetrieschaltung zugeordnet, die ein für die gegenseitige Ausrichtung von Sende- und Empfangsspule kennzeichnendes Signal nach außen liefert. Es ist ferner bei einem solchen Verfahren und einer derartigen Vorrichtung bekannt (Leysieffer et al. "Ein vollständig implantierbares Hörsystem für Innenohrschwerhörige: TICA LZ 3001" in HNO, 1998, 46:853-863), dem Implantatträger Informationen betreffend die Positionierung des Sendeteils gegenüber dem Empfangsteil während des Ladevorganges über akustische Signale zu vermitteln, die in den akustischen Signalpfad der Hörhilfe eingespeist werden. Bei einer anderen bekannten Positionierhilfe eines Ladesystems für implantierbare medizinische Geräte (US-PS 5 690 693) erfolgt an dem Sendeteil eine optische Anzeige der korrekten gegenseitigen Ausrichtung von Sende- und Empfangsteil basierend auf dem durch die Sendespule fließenden Strom.

Es ist Aufgabe der vorliegenden Erfindung, eine Vorrichtung und ein Verfahren zur Unterstützung der manuellen Positionierung des Sendeteils bei einem Ladesystem für implantierbare medizinische Geräte zu schaffen, die zuverlässige und sinnfällige Positioniersignale auf einfache Weise für den Implantatträger bereitstellen können.

Diese Aufgabe wird erfindungsgemäß gelöst durch eine Vorrichtung gemäß Anspruch 1. Entsprechend dem erfindungsgemäßen Verfahren wird im Sendeteil ein mit dem Senderesonanzkreis verbundener Oszillator vorgesehen, dessen Resonanzfrequenz sich in Abhängigkeit von der Kopplung zwischen Sende- und Empfangsspule verschiebt; die Frequenzverstimmung des Oszillators beim Ausrichten des Sendeteils gegenüber dem Empfangsteil wird gemessen, und in Abhängigkeit von der ermittelten Frequenzverstimmung des Oszillators wird ein Positioniersignal ausgegeben, das eine Bewertung der Positionierung erlaubt.

Die Vorrichtung und das Verfahren nach der Erfindung kommen ohne Telemetrie zwischen Sende- und Empfangsteil aus, und sie erlauben es, alle zum Unterstützen der Positionierung erforderlichen Komponenten sendeseitig, das heißt extern, anzuordnen. Sie bleiben selbst dann funktionstüchtig, wenn wegen Erschöpfung der implantierten Energiequelle das implantierte medizinische Gerät oder dessen Telemetrieanordnung ausgefallen sind oder wenn beispielsweise aufgrund einer Ladesstromregelung oder -begrenzung der durch die Sendespule fließenden Strom kein eindeutiges Indiz für das Erreichen einer für den Ladevorgang optimalen Position ist, solange nur die Resonanzeigenschaften des Empfangsresonanzkreis erhalten bleiben.

Prinzipiell geht bei einem Oszillator der vorliegend vorgesehenen Art die Relativbewegung der Sende- und Empfangssspulen in allen drei Raumachsen (X, Y und Z) in die Verstimmung ein. Da für den Ladevorgang der Sendeteil auf die Haut über dem Empfangsteil aufgesetzt wird, ist die Achse Z, welche den Abstand der Spulen zueinander angibt, bei dem Implantatträger durch die Dicke der Haut beziehungsweise des Gewebes über dem Implantat definiert; sie kann für den Positioniervorgang beim individuellen Patienten als konstant angenommen werden.

Vorteilhafte Ausgestaltungen der Erfindung ergeben sich aus den Unteransprüchen. Insbesondere kann der sendeseitige Oszillator einen Donaldson-Oszillator aufweisen. Ein solcher Oszillator ist aus dem Aufsatz von P.E.K. Donaldson "Power For Neurological Prostheses: A Simple Inductive R.F. Link With Improved Performance" in J. Biomed. Eng. Vol. 9, Juli 1987 an sich bekannt. Ein typisches Merkmal eines solchen Oszillators ist es, daß sich seine Resonanzfrequenz in Abhängigkeit von der Kopplung zwischen Sende- und Empfangsspule verschiebt. Die Verschiebung nimmt mit zunehmender Kopplung zwischen Sende- und Empfangskreis zu. Da die Kopplung bei axial ausgerichteten Spulen am größten ist, läßt sich aus dem Maß der Verstimmung die gegenseitige Position der Spulen ableiten. Im Falle eines Donaldson-Oszillators geht in das Maß der Verstimmung auch der momentan im Empfangskreis fließende Ladestrom in der Weise ein, daß mit zunehmendem Ladezustand der wiederaufladbaren Energiequelle die Verstimmung abnimmt. Für die relativ kurze Positionierphase (in der Regel maximal eine Minute) kann das vernachlässigt werden, insbesondere dann, wenn in üblicher Weise jede Ladephase mit dem maximal möglichen Ladestrom beginnt und eine gegebenenfalls vorgesehene Stromregelung erst später einsetzt.

Der Senderesonanzkreis und der Empfangsresonanzkreis sind bevorzugt als Serienresonanzkreise ausgebildet. Vorzugsweise sind ferner die Sendespule und die Empfangsspule zugleich die Spule des Senderesonanzkreises beziehungsweise des Empfangsresonanzkreises.

Für die Nutzung der Frequenzverstimmung des Oszillators als Positionierhilfe soll das Vorzeichen der Verstimmung eindeutig sein. Außerdem ist es bei Kapselung des Implantats in einer Metallhülle, beispielsweise einer Titanhülle, erwünscht, die Verstimmung bei Kopplung der Sendespule zur Empfangsspule von einer Kopplung zu der Metallhülle unterscheiden zu können. Diese Bedingungen lassen sich einfach dadurch erfüllen, daß die Resonanzfrequenz des Senderesonanzkreises von der Resonanzfrequenz des Empfangsresonanzkreises geringfügig, zweckmäßig etwa 0,5 bis 3% , bevorzugt um 1 bis 2%, abweicht, insbesondere tiefer liegt als die Resonanzfrequenz des Empfangsresonanzkreises. Es ergibt sich dann eine negative Frequenzabweichung bei zunehmender Kopplung der Kreise beziehungsweise eine positive Frequenzabweichung bei Kopplung der Sendespule zur Metallhülle des Implantats.

Die Meßanordnung kann zum Ermitteln der Frequenzverstimmung des Oszillators eine die Schwingungen des Oszillators pro Zeiteinheit zählende Zählschaltung aufweisen. Eine solche Zählschaltung kann als diskrete Schaltung aufgebaut oder vorzugsweise in die Funktion eines Mikrokontrollers des Sendeteils integriert sein.

Die Meßanordnung kann zum Bestimmen des Wertes der Frequenzabweichung mit Mitteln zur Messung der Grundfrequenz des Oszillators in ungekoppeltem Zustand und für eine zyklische Vergleichsmessung und Differenzbildung in gekoppeltem Zustand versehen sein. Entsprechend einer abgewandelten Ausgestaltung der Erfindung kann die Meßanordnung mit Mitteln zur Berechnung der Differenz zweier aufeinander folgender Frequenzmeßwerte versehen sein.

Zum Ermitteln der Frequenzverstimmung des Oszillators kann die Meßanordnung aber auch Mittel zum Messen der Periodendauer der Oszillatorschwingungen aufweisen. Die Bestimmung des Wertes der Frequenzabweichung kann dabei in analoger Weise erfolgen. Das heißt, es können Mittel zur Messung der Grundperiodendauer des Oszillators in ungekoppeltem Zustand und für eine zyklische Vergleichsmessung und Differenzbildung in gekoppeltem Zustand vergesehen sein, oder die Meßanordnung kann mit Mitteln zur Berechnung der Differenz zweier aufeinander folgender Periodendauerwerte ausgestattet sein.

Zur Messung der Periodendauer der Oszillatorschwingungen kann eine diskrete Schaltung vorgesehen sein. Vorzugsweise sind jedoch die Mittel zum Messen der Periodendauer der Oszillatorschwingungen in die Funktion eines Mikrokontrollers des Sendeteils integriert.

Die Auswertung der Messungen wird vorzugsweise programmgesteuert in einem Mikrokontroller durchgeführt. Für die Bewertung der Messung und die Entscheidung für ein "gut positioniert - schlecht positioniert" - Signal können in den Mikrokontroller ein oder mehrere Schwellwerte und/oder ein selbst adaptierender Algorithmus programmiert sein.

Die Auswerteanordnung kann insbesondere so ausgelegt sein, daß sie optische und/oder akustische Anzeigesignale abgibt.

Beispielsweise kann zur optischen Signalisierung am Sendeteil eine duo-LED-Einheit vorgesehen sein, die in Abhängigkeit von der jeweiligen gegenseitigen Ausrichtung von Sende- und Empfangsspule z.B. rot, orange oder grün leuchtet.

Für eine akustische Signalisierung kann die Oszillatorfrequenz mit einer festen Referenzfrequenz in der Weise gemischt werden, daß das Mischprodukt einen hörbaren Ton ergibt, wobei die Tonhöhe dieses Tones von der Qualität der Kopplung zwischen Sende- und Empfangsspule abhängt. Die Tonhöhe kann in diesem Fall durch Positionierbewegungen des Sendeteils je nach Schaltungsauslegung auf maximale oder minimale Tonhöhe eingestellt werden.

Im Rahmen der vorliegenden Erfindung werden Messung, Auswertung und Signalisierung in Echtzeit ausgeführt. Das bedeutet, daß der Anwender während der Positionierbewegung als Positionierhilfe eine Rückmeldung, insbesondere eine optische und/oder akustische Rückmeldung, erhält.

Bevorzugte Ausführungsbeispiele der vorliegenden Erfindung werden nachstehend unter Bezugnahme auf die beigefügten Zeichnungen beschrieben. Dabei zeigen:
- Fig. 1: ein schematisches Blockschaltbild einer erfindungsgemäßen Vorrichtung in einer ersten Ausführungsform; und
- Fig. 2: ein schematisches Blockschaltbild einer erfindungsgemäßen Vorrichtung in einer abgewandelten Ausführungsform.

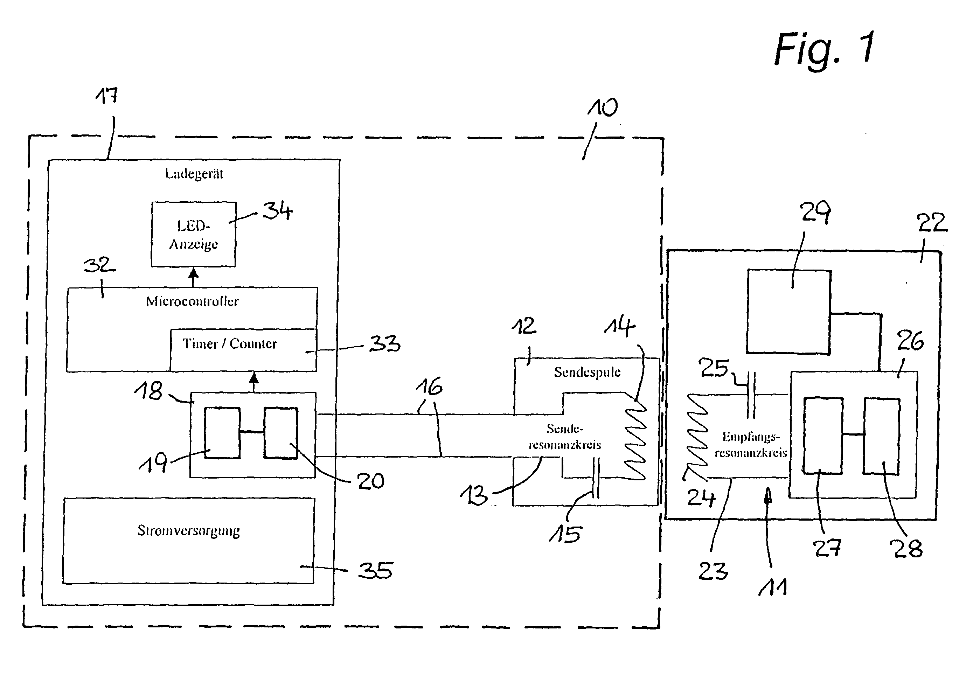

Das in Fig. 1 dargestellte Ladesystem weist einen externen Sendeteil 10 und einen implantierbaren Empfangsteil 11 auf.

Zu dem externen Sendeteil 10 gehört ein Ladekopf 12. In dem Ladekopf 12 ist ein sendeseitiger Serienresonanzkreis 13 mit einer Spule 14 und einem damit in Reihe geschalteten Kondensator 15 untergebracht. Die Spule 14 des Serienresonanzkreises 13 bildet zugleich die Sendespule des Sendeteils 10. Der Ladekopf 12 steht über ein Kabel 16 mit einem Ausgang eines gleichfalls zu dem Sendeteil 10 gehörenden Ladegerätes 17 in Verbindung. Insbesondere ist der Serienresonanzkreis 13 an eine Schaltungsstufe 18 angeschlossen, die einen Donaldson-Oszillator 19 und eine diesem Oszillator nachgeschaltete Sendeendstufe 20 umfaßt.

Der Empfangsteil 11 ist Bestandteil eines insgesamt mit 22 bezeichneten implantierbaren medizinischen Gerätes und weist einen empfangsseitigen Serienresonanzkreis 23 mit einer Spule 24 und einem damit in Reihe geschalteten Kondensator 25 auf. Die Spule 24 des Serienresonanzkreises 23 bildet zugleich die Empfangsspule des Empfangsteils 11. Der Serienresonanzkreis 23 ist an den Eingang einer Schaltungsstufe 26 angeschlossen, zu der eine Laderegelungsschaltung 27 und eine dieser Schaltung nachgeschaltete Energiequelle 28 in Form einer mehrfach wiederaufladbaren Gleichspannungsquelle gehören. Die Energiequelle 28 dient der Energieversorgung eines aktiven medizinischen Implantats 29, insbesondere einer Hörhilfe. Die Energiequelle 28 kann als Energieversorgungsmodul gemäß der älteren EP-Patentanmeldung 98 121 610.4 ausgelegt sein. Die Laderegelungsschaltung 27 und das Ladeverfahren können entsprechend der älteren EP-Patentanmeldung 98 121 498.4 ausgeführt sein. Die Hörhilfe kann für eine direkte mechanische Anregung des Mittel- oder Innenohrs ausgebildet sein. Eine solche Hörhilfe ist beispielsweise aus US-PS 5 277 694, US-PS 5 814095 und dem bereits oben genannten Aufsatz von Leysieffer et al. "Ein vollständig implantierbares Hörsystem für Innenohrschwerhörige: TICA LZ 3001" in HNO, 1998, 46:853-863 bekannt. Die Laderegelungsschaltung 27 und das aktive medizinische Implantat 29 bedürfen daher keiner näheren Erläuterung.

Zu dem Ladegerät 17 gehören ferner ein Mikrokontroller 32 mit einer bei 33 angedeuteten Zeitgeber/Zählstufe, eine an einen Ausgang des Mikrokontrollers 32 angeschlossene LED-Anzeige 34 und eine Stromversorgung 35 für die aktiven Komponenten des Sendeteils 10.

Zum Nachladen der Energiequelle 28 wird der der Ladekopf 12 auf die Haut des Implantatträgers aufgesetzt und manuell so positioniert, daß die Sendespule 14 mit der Empfangsspule 24 möglichst gut axial ausgerichtet ist, so daß die beiden Spulen zusammen den Primär- und den Sekundärkreis eines HF-Übertragers bilden und die Empfangsspule 24 elektromagnetische Energie aus der Sendespule 14 aufnimmt.

Die vorliegend vorgesehene Positionierhilfe nutzt den Umstand, daß sich die Resonanzfrequenz des Donaldson-Oszillators 19 in Abhängigkeit von der induktiven Kopplung zwischen Sende- und Empfangskreis verschiebt, und zwar derart, daß die Verstimmung mit zunehmender Kopplung zwischen Sende- und Empfangsspule 14, 24 zunimmt. Da die Kopplung bei axial ausgerichteten Spulen 14, 24 am größten ist, läßt sich aus dem Maß der Verstimmung die relative Position der Spulen 14, 24 ableiten. Zwar hängt die Verstimmung prinzipiell von der Relativverlagerung der Spulen 14, 24 in allen drei Raumachsen ab. Bei auf die Haut des Implantatträgers aufgesetztem Ladekopf 12 ist aber der Spulenabstand in der einen der drei Raumachsen durch die Dicke der Haut oder des Gewebes über dem Implantat vorgegeben und als konstant anzusehen. In das Maß der Verstimmung geht bei einem Donaldson-Oszillator auch der momentan im Empfangskreis 13 fließende Ladestrom so ein, daß mit zunehmender Aufladung der Energiequelle 28 die Frequenzverstimmung kleiner wird. Da aber die Positionierphase relativ kurz ist (normalerweise maximal 1 Minute), kann für die Positionierung die Ladestromänderung vernachlässigt werden, zumal in der Regel jeder Ladevorgang mit dem maximal möglichen Ladestrom beginnt und die Stromregelung mittels der Laderegelungsschaltung 27 erst in einer späteren Phase des Ladevorgangs einsetzt.

Um für die Nutzung der Frequenzverstimmung des Donaldson-Oszillators 19 als Positionierhilfe ein eindeutiges Vorzeichen der Verstimmung sicherzustellen und auch die Verstimmung bei Kopplung der Sendespule 14 zur Empfangsspule 24 von der Verstimmung bei einer Kopplung der Sendespule 14 zu einer metallischen Hülle des Implantats 22 unterscheiden zu können, ist bei dem beschriebenen Ausführungsbeispiel die Resonanzfrequenz des sendeseitigen Resonanzkreises 13 um 1 bis 2 % tiefer gelegt als die Resonanzfrequenz des empfangsseitigen Resonanzkreises 23. Dies hat zur Folge, daß eine negative Frequenzabweichung bei zunahmender Kopplung der Spulen 14, 24 eintritt, während es zu einer positiven Frequenzabweichung bei Kopplung der Sendespule 14 zu der metallischen Implantathülle kommt.

Die Frequenzverstimmung des Donaldson-Oszillators 19 kann durch eine Zählschaltung ermittelt werden, welche die Schwingungen des Donaldson-Oszillators 19 pro Zeiteinheit zählt. Eine solche Zählschaltung kann als diskrete Schaltung aufgebaut sein. Vorzugsweise ist sie jedoch in die Funktion des Mikrokontrollers 32 integriert. Der Wert der Frequenzabweichung kann ermittelt werden durch
(a) Messung der Grundfrequenz des Oszillators 19 in ungekoppeltem Zustand und zyklische Vergleichsmessung und Differenzbildung bei gekoppelten Spulen 14, 24, oder
(b) Berechnung der Differenz zweier während der Positionierung aufeinanderfolgender Meßwerte (Steigung).

Stattdessen kann die Frequenzverstimmung des Donaldson-Oszillators 19 auch durch eine Messung der Periodendauer der Oszillatorschwingungen ermittelt werden. Dazu kann eine diskrete Schaltung vorgesehen sein. Vorzugsweise ist eine derartige Periodendauermessung aber in die Funktion des Mikrokontrollers 32 integriert. Der Wert der Frequenzabweichung kann analog den vorstehenden Alternativen (a) und (b) bestimmt werden.

Die zuvor erläuterten Messungen werden vorzugsweise programmgesteuert in dem Mikrokontroller 32 ausgewertet. Insbesondere können für die Bewertung der Messung und eine Entscheidung für ein Positioniersignal, das eine gute oder eine schlechte Positionierung signalisiert, ein oder mehrere Schwellenwerte und/oder ein selbst adaptierender Algorithmus in einem Programmspeicher des Mikrokontrollers 32 programmiert sein.

Bei dem Ausführungsbeispiel der Fig. 1 steuert der Mikrokontroller 32 die LED-Anzeige 34 entsprechend der zuvor genannten Auswertung an. Bei der LED-Anzeige 34 kann es sich beispielsweise um eine duo-LED-Anzeigeinheit handeln, die in Abhängigkeit von dem Ansteuersignal des Mikrokontrollers 32 rot, orange oder grün leuchtet und so den Implantatträger leicht erkennen läßt, wenn der Ladekopf 12 eine Stellung einnimmt, in der eine befriedigende Kopplung der Spulen 14 und 24 herbeigeführt ist. Es versteht sich, daß auch andere optische Anzeigeeinheiten vorgesehen werden können. Ferner kann die Positionserkennung statt durch einen Farbwechsel der Anzeige oder zusätzlich dazu auch durch eine zeitlich differenzierte Ansteuerung (Blinken) erfolgen.

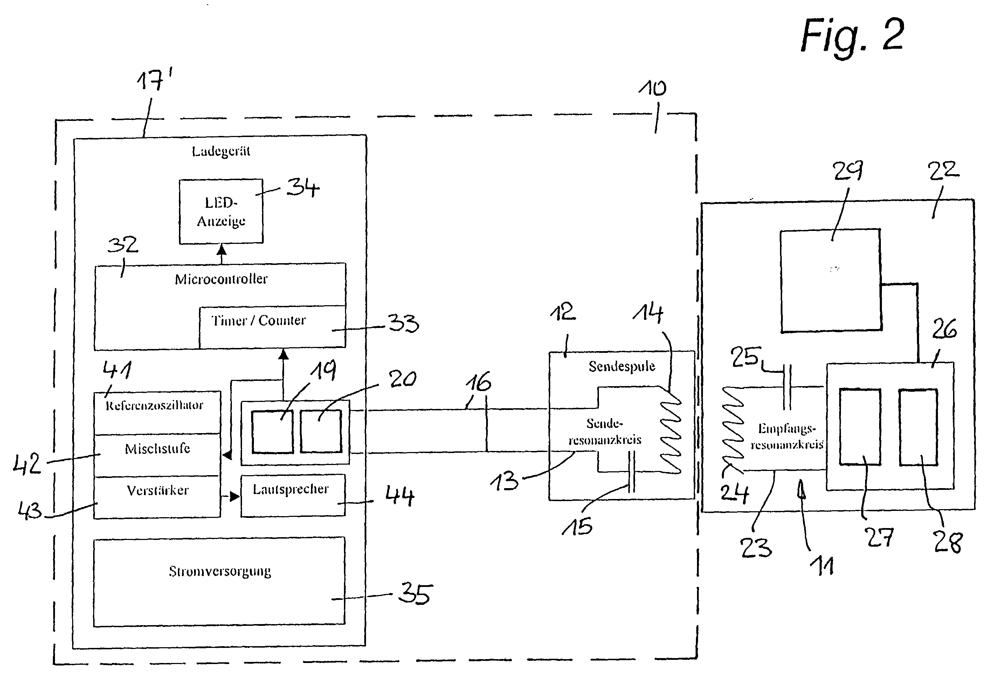

Das Ausführungsbeispiel der Fig. 2 unterscheidet sich von dem der Fig. 1 dadurch, daß ein Ladegerät 17' vorgesehen ist, das zusätzlich einen Referenzoszillator 41, eine Mischstufe 42, einen Verstärker 43 und einen Lautsprecher 44 aufweist.

Der Referenzoszillator 41 erzeugt eine feste Referenzfrequenz, die in der Mischstufe 42 mit der Frequenz des Donaldson-Oszillators 19 in der Weise gemischt wird, daß als Mischprodukt ein Signal im hörbaren Frequenzbereich entsteht. Dieses Signal wird in dem Verstärker 43 verstärkt und von dem Lautsprecher 44 als hörbarer Ton abgegeben. Die Tonhöhe ist dabei von der Qualität der Kopplung der Spulen 14, 24 abhängig und kann damit als Indikator für die Position des Ladekopfes 12 relativ zu dem Implantat 10 benutzt werden. Durch Positionierbewegungen der Sendespule 14 wird die Tonhöhe je nach Schaltungsauslegung auf Maximum beziehungsweise Minimum eingestellt.

## Patentansprüche

1. Vorrichtung mit einem externen Sendeteil (10) und einem implantierbaren Empfangsteil (11) eines Ladesystems zum Aufladen einer wiederaufladbaren Energiequelle (28) eines implantierbaren medizinischen Gerätes (22), insbesondere einer implantierbaren Hörhilfe, wobei der Sende- und der Empfangsteil jeweils einen Resonanzkreis (13, 23) mit einer Sendespule (14) beziehungsweise einer Empfangsspule (24) aufweisen und wobei diese Spulen durch entsprechendes manuelles Positionieren des Sendeteils für eine transkutane Energieübertragung induktiv miteinander koppelbar sind, wobei der Sendeteil (10) einen mit dem Senderesonanzkreis (13) verbundenen Oszillator (19) sowie eine Auswerteanordnung (32) aufweist, von der ein von der Kopplung zwischen Sende- und Empfangsteil abhängiges Positioniersignal ausgebbar ist, **dadurch gekennzeichnet, daß** die Resonanzfrequenz des Oszillators sich in Abhängigkeit von der Kopplung zwischen Sende- und Empfangsspule (14, 24) verschiebt, daß eine Meßanordnung (32) zum Bestimmen der Frequenzverstimmung des Oszillators vorgesehen ist und dass von der Auswerteanordnung (32) das Positioniersignal in Abhängigkeit von der ermittelten Frequenzverstimmung des Oszillators ausgebbar ist.

2. Vorrichtung nach Anspruch 1, **dadurch gekennzeichnet, daß** der Senderesonanzkreis (13) und der Empfangsresonanzkreis (23) als Serienresonanzkreise ausgebildet sind.

3. Vorrichtung nach einem der vorhergehenden Ansprüche, **dadurch gekennzeichnet, daß** die Sendespule (14) zugleich die Spule des Senderesonanzkreises (13) ist.

4. Vorrichtung nach einem der vorhergehenden Ansprüche, **dadurch gekennzeichnet, daß** die Empfangsspule (24) zugleich die Spule des Empfangsresonanzkreises (23) ist.

5. Vorrichtung nach einem der vorhergehenden Ansprüche, **dadurch gekennzeichnet, daß** die Resonanzfrequenz des Senderesonanzkreises (13) von der Resonanzfrequenz des Empfangsresonanzkreises (23) geringfügig abweicht.

6. Vorrichtung nach Anspruch 5, **dadurch gekennzeichnet, daß** die Resonanzfrequenz des Senderesonanzkreises (13) tiefer liegt als die Resonanzfrequenz des Empfangsresonanzkreises (23).

7. Vorrichtung nach Ansprüchen 5 oder 6, **dadurch gekennzeichnet, daß** die Resonanzfrequenz des Senderesonanzkreises (13) von der Resonanzfrequenz des Empfangsresonanzkreises (23) um 0,5 bis 3% , bevorzugt um 1 bis 2%, abweicht.

8. Vorrichtung nach einem der vorhergehenden Ansprüche, **dadurch gekennzeichnet, daß** die Meßanordnung (32) eine die Schwingungen des Oszillators (19) pro Zeiteinheit zählende Zählschaltung aufweist.

9. Vorrichtung nach Anspruch 8, **dadurch gekennzeichnet, daß** der Sendeteil (10) einen Mikrokontroller (32) aufweist, der derart programmiert ist, daß er die Zählschaltung aufweist.

10. Vorrichtung nach Anspruch 8 oder 9, **dadurch gekennzeichnet, daß** die Meßanordnung (32) mit Mitteln zur Messung der Grundfrequenz des Oszillators (19) in ungekoppeltem Zustand und für eine zyklische Vergleichsmessung und Differenzbildung in gekoppeltem Zustand versehen ist.

11. Vorrichtung nach Anspruch 8 oder 9, **dadurch gekennzeichnet, daß** die Meßanordnung (32) mit Mitteln zur Berechnung der Differenz zweier aufeinander folgender Frequenzmeßwerte versehen ist.

12. Vorrichtung nach einem der Ansprüche 1 bis 7, **dadurch gekennzeichnet, daß** die Meßanordnung (32) Mittel zum Messen der Periodendauer der Oszillatorschwingungen aufweist.

13. Vorrichtung nach Anspruch 12, **dadurch gekennzeichnet, daß** der Sendeteil (10) einen Mikrokontroller (32) aufweist, der derart programmiert ist, daß er die Mittel zum Messen der Periodendauer der Oszillatorschwingungen aufweist.

14. Vorrichtung nach Anspruch 12 oder 13, **dadurch gekennzeichnet, daß** die Meßanordnung (32) mit Mitteln zur Messung der Grundperiodendauer der Oszillatorschwingungen in ungekoppeltem Zustand und für eine zyklische Vergleichsmessung und Differenzbildung in gekoppeltem Zustand versehen ist.

15. Vorrichtung nach Anspruch 12 oder 13, **dadurch gekennzeichnet, daß** die Meßanordnung (32) mit Mitteln zur Berechnung der Differenz zweier aufeinander folgender Periodendauer-Meßwerte versehen ist.

16. Vorrichtung nach einem der vorhergehenden Ansprüche, **dadurch gekennzeichnet, daß** der Sendeteil (10) einen Mikrokontroller (32) aufweist, der derart programmiert ist, daß er die Auswerteanordnung aufweist.

17. Vorrichtung nach Anspruch 16, **dadurch gekennzeichnet, daß** die in den Mikrokontroller (32) programmierte Auswertung einen oder mehrere Schwellwerte und/oder einen selbst adaptierenden Algorithmus aufweist.

18. Vorrichtung nach einem der vorhergehenden Ansprüche, **dadurch gekennzeichnet, daß** die Auswerteanordnung (32) Signale für eine optische und/oder akustische Anzeige abgibt.

19. Verfahren zum Unterstützen der Positionierung eines externen Sendeteils mit Bezug auf ein implantierbares Empfangsteil eines Ladesystems zum Aufladen einer wiederaufladbaren Energiequelle eines implantierbaren medizinischen Gerätes, insbesondere einer implantierbaren Hörhilfe, wobei der Sende- und der Empfangsteil jeweils einen Resonanzkreis mit einer Sendespule beziehungsweise einer Empfangsspule aufweisen und wobei diese Spulen durch entsprechendes manuelles Positionieren des Sendeteils für eine transkutane Energieübertragung induktiv miteinander gekoppelt werden, wobei im Sendeteil ein mit dem Senderesonanzkreis verbundener Oszillator vorgesehen wird und ein von der Kopplung zwischen Sende- und Empfangsteil abhängiges Positioniersignal ausgegeben wird, **dadurch gekennzeichnet, daß** die Resonanzfrequenz des Oszillators sich in Abhängigkeit von der Kopplung zwischen Sende- und Empfangsspule verschiebt, daß die Frequenzverstimmung des Oszillators beim Ausrichten des Sendeteils gegenüber dem Empfangsteil gemessen wird, und daß in Abhängigkeit von der ermittelten Frequenzverstimmung des Oszillators das Positioniersignal ausgegeben wird, das eine Bewertung der Positionierung erlaubt.

20. Verfahren nach Anspruch 19, **dadurch gekennzeichnet, daß** zum Ermitteln der Frequenzverstimmung die Schwingungen des Oszillators pro Zeiteinheit in ungekoppeltem Zustand und in gekoppeltem gezählt und miteinander verglichen werden.

21. Verfahren nach Anspruch 19, **dadurch gekennzeichnet, daß** zum Ermitteln der Frequenzverstimmung die Periodendauern der Oszillatorschwingungen in ungekoppeltem Zustand und in gekoppeltem Zustand gezählt und miteinander verglichen werden.

22. Verfahren nach einem der Ansprüche 19 bis 21, **dadurch gekennzeichnet, daß** ein von der ermittelten Frequenzverstimmung abhängiges optisches und/oder akustisches Signal als Positionierhilfesignal ausgegeben wird.

23. Verfahren nach Anspruch 22, **dadurch gekennzeichnet, daß** die Oszillatorfrequenz mit einer festen Referenzfrequenz in der Weise gemischt wird, daß das Mischprodukt einen hörbaren Ton ergibt, wobei die Tonhöhe dieses Tones von der Qualität der Kopplung zwischen Sende- und Empfangsspule abhängt, und daß durch Positionierbewegungen des Sendeteils auf maximale oder minimale Tonhöhe eingestellt wird.

## Claims

1. Device comprising an external transmitting part (10) and an implantable receiving part (11) of a charging system for charging a rechargeable power source (28) of an implantable medical device, in particular an implantable hearing aid, wherein the transmitting part and the receiving part each comprise a resonant circuit (13, 23) having a transmitting coil (14) and a receiving coil (24), respectively, and wherein said coils can be coupled inductively to each other by respective manual positioning of the transmitting part for transcutaneous power transmission, wherein the transmitting part comprises an oscillator (19) connected to the transmitting resonant circuit (13) and an evaluating arrangement (32) which is adapted to output a positioning signal depending on the coupling between the transmitting part and the receiving part, **characterized in that** the resonant frequency of the oscillator detunes depending on the coupling between the transmitting coil (14) and the receiving coil (24), that a measurement arrangement for determining the frequency detuning of the oscillator is provided and that the evaluation arrangement (32) is adapted to output the positioning signal depending on the determined frequency detuning of the oscillator.

2. Device according to claim 1, **characterized in that** said transmitting resonant circuit (13) and said receiving resonant circuit (23) are series resonant circuits.

3. Device according to one of the preceding claims, **characterized in that** the transmitting coil (14) also functions as the coil of the transmitting resonant circuit.

4. Device according to one of the preceding claims, **characterized in that** the receiving coil (24) also functions as the coil of the resonant receiving circuit (23).

5. Device according to one of the preceding claims, **characterized in that** the resonant frequency of said transmitting resonant circuit (13) differs slightly from the resonant frequency of said receiving resonant circuit (23).

6. Device according to claim 5, **characterized in that** the resonant frequency of said transmitting resonant circuit (13) is lower than the resonant frequency of said receiving resonant circuit (23).

7. Device according to claim 5 or 6, **characterized in that** the resonant frequency of said transmitting resonant circuit (13) differs from the resonant frequency of said receiving resonant circuit (23) between 0.5 to 3 percent, preferably 1 to 2 percent.

8. Device according to one of the preceding claims, **characterized in that** the measurement arrangement (32) comprises a counting circuit which counts the oscillations of the oscillator (19) per time unit.

9. Device according to claim 8, **characterized in that** the transmitting part (10) comprises a microcontroller (32) which is programmed such that it comprises the counting circuit.

10. Device according to claims 8 or 9, **characterized in that** said measurement arrangement (32) includes means for measuring the fundamental frequency of said oscillator (19) in an uncoupled state and for cyclic comparison measurement and difference formation in a coupled state.

11. Device according to claims 8 or 9, **characterized in that** said measurement arrangement (32) includes means for computing the difference between two succeeding frequency measurement values.

12. Device according to one of claims 1 to 7, **characterized in that** the measurement arrangement (32) includes means for measuring the period of the oscillator oscillations.

13. Device according to claim 12, **characterized in that** the transmitting part (10) comprises a microcontroller (32) which is programmed such that it comprises the means for measuring the period length of the oscillator vibrations.

14. Device according to claim 12 or 13, **characterized in that** said measurement arrangement (32) includes means for measuring the basic period of the oscillator oscillations in an uncoupled state and for cyclic comparison measurement and difference formation in a coupled state.

15. Device according to claim 12 or 13, **characterized in that** said measurement arrangement (32) includes means for computing the difference between two succeeding period measurement values.

16. Device according to one of the preceding claims, **characterized in that** the transmitting part (10) comprises a microcontroller (32) which is programmed such that it comprises the evaluation arrangement.

17. Device according to claim 16, **characterized in that** the evaluation programmed in the microcontroller (32) comprises at least one threshold value and/or at least one self-adapting algorithm.

18. Device according to one of the preceding claims, **characterized in that** said evaluation arrangement (32) delivers signals for an optical display and/or an acoustic speaker.

19. Method for aiding the positioning of an external transmitting part relative to an implantable receiving part of a charging system for charging a rechargeable power source of an implantable medical device, in particular an implantable hearing aid, wherein the transmitting part and the receiving part each comprise a resonant circuit having a transmitting coil and a receiving coil, respectively, and wherein said coils are coupled inductively to each other by corresponding manual positioning of the transmitting part for a transcutaneous power transmission, wherein an oscillator connected to the transmitting resonance circuit is provided in the transmitting part, and wherein a positioning signal dependent on the coupling between the transmitting part and the receiving part is output, **characterized in that** the resonant frequency of the oscillator detunes depending on the coupling between the transmitting coil and the receiving coil, that the frequency detuning of the oscillator is measured during positioning of the transmitting part relative to the receiving part, and that the positioning signal is output depending on the measured frequency detuning of the oscillator for enabling an evaluation of the positioning.

20. Method according to claim 19, **characterized in that** for determining the frequency detuning the oscillations per unit of time are counted and compared in the uncoupled state and in the coupled state.

21. Method according to claim 19, **characterized in that** for determining the frequency detuning the period of the oscillator oscillations are counted and compared in the uncoupled state and in the coupled state.

22. Method according to one of claims 19 to 21, **characterized in that** an optical signal and/or an acoustic signal which is dependent on the determined frequency detuning is output as a positioning aid signal.

23. Method according to claim 22, **characterized in that** the oscillator frequency is mixed with a fixed reference frequency in a manner that the resulting mixed product yields an audible tone, wherein the frequency of this tone depends on the quality of the inductive coupling between said transmitting coil and **in that** adjustment takes place by positioning movements of said transmitting coil relative to said receiving coil for achieving maximum or minimum frequency.

## Revendications

1. Dispositif avec un élément émetteur externe (10) et un élément récepteur implantable (11) d'un système de charge pour charger une source d'énergie rechargeable (28) d'un appareil médical implantable (22), en particulier d'une prothèse auditive implantable, l'élément émetteur et l'élément récepteur présentant chacun un circuit résonant (13, 23) avec une bobine émettrice (14) respectivement une bobine réceptrice (24) et ces bobines pouvant être couplées l'une à l'autre de manière inductive par un positionnement manuel correspondant de l'élément émetteur pour une transmission transcutanée d'énergie, l'élément émetteur (10) présentant un oscillateur (19) relié au circuit résonant de l'émetteur (13) ainsi qu'un dispositif d'exploitation (32), par lequel peut être émis un signal de positionnement en fonction du couplage entre l'élément émetteur et l'élément récepteur, **caractérisé en ce que** la fréquence de résonance de l'oscillateur fluctue en fonction du couplage entre la bobine émettrice et la bobine réceptrice (14, 24), **en ce qu'**il est prévu un dispositif de mesure (32) pour déterminer le désaccord de fréquence de l'oscillateur et **en ce que** le signal de positionnement peut être émis par le dispositif d'exploitation (32) en fonction du désaccord de fréquence déterminé de l'oscillateur.

2. Dispositif selon la revendication 1, **caractérisé en ce que** le circuit résonant de l'émetteur (13) et le circuit résonant du récepteur (23) sont formés par des circuits résonants en série.

3. Dispositif selon l'une des revendications précédentes, **caractérisé en ce que** la bobine émettrice (14) est en même temps la bobine du circuit résonant de l'émetteur (13).

4. Dispositif selon l'une des revendications précédentes, **caractérisé en ce que** la bobine réceptrice (24) est en même temps la bobine du circuit résonant du récepteur (23).

5. Dispositif selon l'une des revendications précédentes, **caractérisé en ce que** la fréquence de résonance du circuit résonant de l'émetteur (13) diffère légèrement de la fréquence de résonance du circuit résonant du récepteur (23).

6. Dispositif selon la revendication 5, **caractérisé en ce que** la fréquence de résonance du circuit résonant de l'émetteur (13) est inférieure à la fréquence de résonance du circuit résonant du récepteur (23).

7. Dispositif selon la revendication 5 ou 6, **caractérisé en ce que** la fréquence de résonance du circuit résonant de l'émetteur (13) diffère de 0,5 à 3 %, de préférence de 1 à 2 %, de la fréquence de résonance du circuit résonant du récepteur (23).

8. Dispositif selon l'une des revendications précédentes, **caractérisé en ce que** le dispositif de mesure (32) comporte un circuit de comptage comptant les oscillations de l'oscillateur (19) par unité de temps.

9. Dispositif selon la revendication 8, **caractérisé en ce que** l'élément émetteur (10) comprend un microcontrôleur (32), qui est programmé de façon à présenter le circuit de comptage.

10. Dispositif selon la revendication 8 ou 9, **caractérisé en ce que** le dispositif de mesure (32) est pourvu de moyens pour mesurer la fréquence de base de l'oscillateur (19) à l'état non couplé et pour effectuer une mesure de comparaison et une formation de différence cycliques à l'état couplé.

11. Dispositif selon la revendication 8 ou 9, **caractérisé en ce que** le dispositif de mesure (32) est pourvu de moyens pour calculer la différence entre deux valeurs de mesure successives de la fréquence.

12. Dispositif selon l'une des revendications 1 à 7, **caractérisé en ce que** le dispositif de mesure (32) comporte des moyens pour mesurer la durée de la période des oscillations de l'oscillateur.

13. Dispositif selon la revendication 12, **caractérisé en ce que** l'élément émetteur (10) comprend un microcontrôleur (32), qui est programmé de telle façon qu'il présente les moyens pour mesurer la durée de la période des oscillations de l'oscillateur.

14. Dispositif selon la revendication 12 ou 13, **caractérisé en ce que** le dispositif de mesure (32) est pourvu de moyens pour mesurer la durée de la période de base des oscillations de l'oscillateur à l'état non couplé et pour effectuer une mesure de comparaison et une formation de différence cycliques à l'état couplé.

15. Dispositif selon la revendication 12 ou 13, **caractérisé en ce que** le dispositif de mesure (32) est pourvu de moyens pour calculer la différence entre deux valeurs de mesure successives de la durée de la période.

16. Dispositif selon l'une des revendications précédentes, **caractérisé en ce que** l'élément émetteur (10) comporte un microcontrôleur (32), qui est programmé de façon à présenter le dispositif d'exploitation.

17. Dispositif selon la revendication 16, **caractérisé en ce que** l'exploitation programmée dans le microcontrôleur (32) présente une ou plusieurs valeurs de seuil et/ou un algorithme auto-adaptatif.

18. Dispositif selon l'une des revendications précédentes, **caractérisé en ce que** le dispositif d'exploitation (32) émet des signaux pour un affichage optique et/ou acoustique.

19. Procédé d'aide au positionnement d'un élément émetteur externe par rapport à un élément récepteur implantable d'un système de charge pour charger une source d'énergie rechargeable d'un appareil médical implantable, en particulier d'une prothèse auditive implantable, l'élément émetteur et l'élément récepteur présentant chacun un circuit résonant avec une bobine émettrice ou une bobine réceptrice et ces bobines étant couplées de manière inductive l'une à l'autre par un positionnement manuel correspondant de l'élément émetteur pour une transmission transcutanée d'énergie, un oscillateur relié au circuit résonant étant prévu dans l'élément émetteur et un signal de positionnement étant émis en fonction du couplage entre l'élément émetteur et l'élément récepteur, **caractérisé en ce que** la fréquence de résonance de l'oscillateur fluctue en fonction du couplage entre la bobine émettrice et la bobine réceptrice, **en ce que** l'on mesure le désaccord de fréquence de l'oscillateur lors de l'orientation de l'élément émetteur par rapport à l'élément récepteur, et **en ce que** l'on émet en fonction du désaccord de fréquence déterminé de l'oscillateur le signal de positionnement qui permet une évaluation du positionnement.

20. Procédé selon la revendication 19, **caractérisé en ce que**, pour déterminer le désaccord de fréquence, on compte et on compare les unes aux autres les oscillations de l'oscillateur par unité de temps à l'état non couplé et à l'état couplé.

21. Procédé selon la revendication 19, **caractérisé en ce que**, pour déterminer le désaccord de fréquence, on compte et on compare les unes aux autres les durées de période des oscillations de l'oscillateur à l'état non couplé et à l'état couplé.

22. Procédé selon l'une des revendications 19 à 21, **caractérisé en ce que** l'on émet un signal optique et/ou acoustique, en fonction du désaccord de fréquence déterminé, comme signal d'aide au positionnement.

23. Procédé selon la revendication 22, **caractérisé en ce que** l'on mélange la fréquence de l'oscillateur avec une fréquence de référence fixe de telle façon que le produit du mélange fournisse une tonalité audible, la hauteur de son de cette tonalité dépendant de la qualité du couplage entre la bobine émettrice et la bobine réceptrice, et **en ce que** l'on règle le son à une hauteur maximale ou minimale par des mouvements de positionnement de l'élément émetteur.
